# EUROPEAN PATENT SPECIFICATION

(11) **EP 2 554 105 B1**
(45) Date of publication and mention of the grant of the patent: **26.11.2014**
(21) Application number: 10848877.6
(22) Date of filing: 28.12.2010
(51) Int. Cl.: A61B 1/06, A61B 1/00, A61B 8/12

(54) **PROBE AND IMAGE DIAGNOSIS DEVICE**
SONDE UND BILDDIAGNOSEVORRICHTUNG
SONDE ET DISPOSITIF DE DIAGNOSTIC D'IMAGE

(30) Priority: 30.03.2010 JP 2010079583
(43) Date of publication of application: 06.02.2013
(73) Proprietor: Terumo Kabushiki Kaisha, Tokyo 151-0072 (JP)
(72) Inventor: MITSUHASHI, Kenta, Fujinomiya-shi Shizuoka 418-0015 (JP); NAKAMOTO, Ryou, Kanaga-wa 259-0151 (JP)
(74) Representative: Dossmann, Gérard
(86) International application number: PCT/JP2010/007594
(87) International publication number: WO 2011/121695

(56) References cited:
- JP-A- 1 250 919
- JP-A- 3 275 030
- JP-A- S5 766 731
- JP-A- 57 066 731
- JP-A- 2003 325 543
- JP-A- 2009 240 711
- US-A- 4 030 494
- US-A- 5 352 210
- US-A- 5 431 641
- US-A1- 2003 216 723

## Description

### TECHNICAL FIELD

The present invention relates to a probe and an imaging apparatus for diagnosis.

### BACKGROUND ART

In the past, there have been widely utilized an optical coherent tomography imaging apparatus for diagnosis (OCT) (for example, see Patent Document 1) or an optical frequency domain imaging (OFDI) apparatus utilizing wavelength sweep, which is an improved type of that above, for a diagnosis before operation at the time of treatment inside a blood vessel depending on a high functional catheter such as a balloon catheter, a stent or the like, or for a result confirmation after operation (hereinafter, it sometimes happens that the optical coherent tomography imaging apparatus for diagnosis (OCT) and the optical frequency domain imaging (OFDI) apparatus utilizing wavelength sweep are generically referred to as "imaging apparatus for diagnosis").

In the imaging apparatus for diagnosis, there is attached an optical probe unit to which an optical lens and an optical mirror (transmitting & receiving unit) are attached. On an occasion of measurement (for example, diagnosis), the optical probe unit is inserted inside the blood vessel and while rotating the imaging core by a scanner & pull-back unit (adopter apparatus), the measurement light is emanated from the transmitting & receiving unit at the distal end into the blood vessel and concurrently, reflected light from the biological tissue is light-received. Thus, a radial scan inside the blood vessel is carried out. Then, interference light is generated by making the aforementioned light-received reflected-light and the reference light interfere with each other and a tomographic image of the blood vessel is visualized based on the aforementioned interference light. Note that for the imaging apparatus for diagnosis, there is also known an apparatus of a system using ultra-sound or the like other than the system using light mentioned above.

The optical probe unit is provided with a connector to be connected to the scanner & pull-back unit. In the optical probe unit, there is received a signal (light, electrical signal) from the scanner & pull-back unit through this connector. Therefore, it is necessary to prevent this connector from becoming dirty caused by a phenomenon in which a foreign matter such as liquid (for example, blood, saline), dust or the like is attached, or the like.

Here, in case of a situation in which the connector has become dirty and has become wet due to foreign matters such as liquid of blood, saline or the like, dust, and the like, there is a possibility that problems such as follows and the like are caused.
1. Electric Shock to Patient
2. Contamination of Optical Probe Unit
3. Rust and/or Corrosion of Scanner & Pull-back Unit
4. Attenuation of Signal (Optical Loss, etc.)

### Prior-art Document

### Patent Document

Patent Document 1: Japanese unexamined patent publication No. 2005-196080

US 2003/216723 A1 discloses an operation system and a connecting adapter. The operation system includes an operation microscope, an external device connectable to the operation microscope, and a connecting adapter for electrically connecting the operation microscope and the external device without bringing the operation microscope and the external device into contact with each other, which maintains a state of sterilization of the external device.

JP S57 66731 A shows a probe with a connector having
- a second cover which is constituted movably toward a second direction expressing the direction opposite to a first direction expressing the direction toward which the probe is inserted. JP S57 66731 A is silent on the rightward-side end surface of the second cover.

US 5 431 641 A discloses a plug-type connection that is used for connecting at least one sterile electrical, optical and/or fluid-carrying line to at least one non-sterile apparatus with one line-side and one equipment-side plug unit respectively. The equipment-side plug unit is surrounded by at least one sterile foil which is clamped tightly between respective corresponding sealing and clamping edges of the line-side and the equipment-side plug units which surround the line to be plugged in. The line-side and/or equipment-side plug unit has a cutting device to cut the foil in the area of the lines to be contacted.

US 5 352 210 A discloses one of the two confined volumes, for example the "supplying" one (2), is provided with a tubular perforating element (3) confined within a space closed by a diaphragm or perforable wall (503); the second confined volume (1) also has a perforable diaphragm (301); the surfaces of the two diaphragms (503, 301) directed towards the external environment are permanently bonded together after which the perforator (3) is manipulated from the outside so as to perforate the two diaphragms, placing in communication, via the tubular duct of the perforator, the confined environment of the supplying volume with that of the second volume.

US 4 030 494 A discloses a connector means for conveying fluid from a supply source to a delivery location, said connectors having one conduit extending to the supply source and another conduit extending to the delivery location. Each terminal part has a cylindrical housing with a closed end to which the conduit is joined, and having an opposite operable end which is closed by a penetrable barrier. The barrier and closed end of the housing define an interior environment safe from external contaminants. A male tubular coupler is in one housing, a female tubular coupler is in the other housing, and a penetrator element is disposed around one of the tubular connectors. The penetrable barriers have a film of covered adhesive. The exposed adhesive films are placed in face to face contact to coaxially align the housing parts and bond the barrier membranes to exclude the environment. The penetrator element pierces the adhering barriers by either moving the penetrator element from one housing into the coaxially aligned housing, or by telescoping the housing cylinders so that the adhering barriers are contacted by the fixed penetrator element. The male tubular connector then engages the female tubular connector within a protected environment to effect connection between the supply source and the delivery location.

### DISCLOSURE OF THE INVENTION

### [Problem to be solved by the Invention]

In order to deal with that problem, for example, there is known a technology in which the connection end surface of the connector is protected by using a cap or the like which is formed by plastic, a flexible resin or the like. However, even if protecting the connection end surface by using such a cap, if the operator's hand becomes wet or the like caused by saline, blood or the like when the operator disengages the cap, there is a possibility that the connection end surface of the connector is to be made wet.

More specifically, even if the connection end surface of the connector is protected by a cap, it is possible for the operator to touch the connector easily, so that there is a risk in which there still occurs a phenomenon in which the connection end surface will get dirty.

Consequently, the present invention was invented in view of the abovementioned problem and is addressed to provide such a technology that the operator is prevented from easily touching the connector and there are prevented phenomena in which the aforementioned connector will become wet, become dirty and so on.

### [Means for solving the Problem]

In order to solve the abovementioned problem, the present invention provides a probe according to independent claim 1 as well as a an imaging apparatus for diagnosis according to co-dependent claim 4. The dependent claims relate to advantageous embodiments.

### [Effect of the Invention]

According to the present invention, it is not easy for the operator to touch the connector. Therefore, it is possible to prevent the connector from becoming wet, from becoming dirty and so on.

Other features and advantages of the present invention will become clear from the following explanation with reference to the attached drawings. Note that in the attached drawings, the same reference numerals are applied for the same or similar constitutions.

### BRIEF DESCRIPTION OF DRAWINGS

The attached drawings are included in the specification, constitute a portion thereof, show exemplified embodiments of the present invention and are used together with the description thereof for explaining the principle of the present invention.
FIG. 1 is a view showing one example of an exterior constitution of an imaging apparatus for diagnosis 10 relating to one exemplified embodiment of the present invention;
FIG. 2 is a view showing one example of a constitution of an optical probe unit 11 shown in FIG. 1;
FIG. 3 is a view showing one example of an outlined constitution of a connection connector unit 30 shown in FIG. 2;
FIG. 4 is a view showing one example of an outlined constitution of the connection connector unit 30 shown in FIG. 2;
FIG. 5A is a view showing one example of a modified example of the connection connector unit 30 relating to an exemplified embodiment 1;
FIG. 5B is a view showing one example of a modified example of the connection connector unit 30 relating to the exemplified embodiment 1;
FIG. 6A is a view showing one example of a modified example of the connection connector unit 30 relating to the exemplified embodiment 1;
FIG. 6B is a view showing one example of a modified example of the connection connector unit 30 relating to the exemplified embodiment 1;
FIG. 7A is a view showing one example of an outlined constitution of the connection connector unit 30 relating to an exemplified

### embodiment 2; and

FIG. 7B is a view showing one example of an outlined constitution of the connection connector unit 30 relating to the exemplified embodiment 2.

### MODE FOR CARRYING OUT THE INVENTION

Hereinafter, there will be explained preferable exemplified embodiments of the present invention in detail with reference to the drawings.

### (Exemplified Embodiment 1)

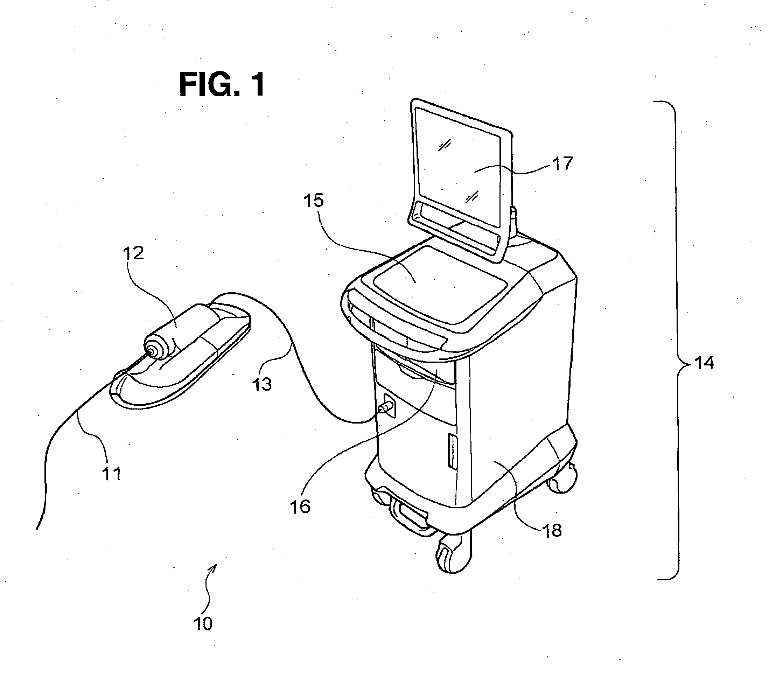

FIG. 1 is a view showing one example of an exterior constitution of an imaging apparatus for diagnosis (optical coherent tomography imaging apparatus for diagnosis or optical frequency domain imaging apparatus utilizing wavelength sweep) 10 relating to one exemplified embodiment of the present invention.

The imaging apparatus for diagnosis 10 is constituted by including an optical probe unit 11, a scanner & pull-back unit (adopter apparatus) 12 and an operation control apparatus 14. The scanner & pull-back unit 12 and the operation control apparatus 14 are connected by means of a signal line 13.

The optical probe unit 11 is inserted inside a body-cavity such as a blood vessel or the like and measures the state inside the body-cavity. The scanner & pull-back unit 12 is constituted detachably with respect to the optical probe unit 11 and controls the radial operation of the imaging core inserted inside the optical probe unit 11 by a mechanism that a built-in motor is driven. The scanner & pull-back unit 12 is provided with an adapter unit (adapter unit 40 described later), and gives and receives various kinds of signals with respect to the optical probe unit 11 through the aforementioned adapter unit.

The operation control apparatus 14 is provided with a function for inputting various kinds of set values and a function for processing data obtained by the measurement and for displaying them as tomographic images.

Here, the operation control apparatus 14 is constituted by including a main body control unit 18, a printer & DVD recorder 16 and an operation panel 15. The main body control unit 18 processes data obtained by the measurement, outputs the process result thereof and so on. The printer & DVD recorder 16 prints the process result in the main body control unit 18, stores it as data and so on. The operation panel 15 inputs various kinds of instructions from the operator to the inside of the apparatus. More specifically, the operator inputs various kinds of set values and instructions through the operation panel 15. LCD monitor 17 is a display apparatus and displays various kinds of screens for the operator. For example, the process result in the main body control unit 18 is displayed for the operator.

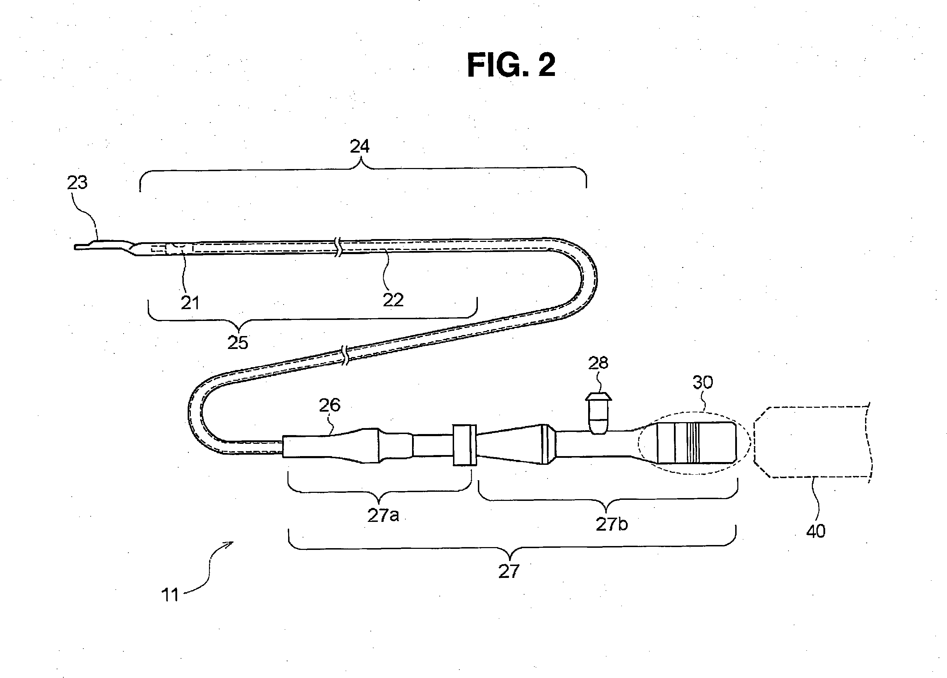

Next, by using FIG. 2, there will be explained one example of a constitution of the optical probe unit 11 shown in FIG. 1.

The optical probe unit 11 is constituted by a long-sized catheter sheath 24 to be inserted inside a body-cavity such as a blood vessel and the like, and a connector unit 27 which is arranged on the hand-side of a user without being inserted inside the body-cavity (in order to be operated by a user). At the distal end of the catheter sheath 24, there is formed a tube 23 for a guide wire lumen, and the catheter sheath 24 is formed as a lumen which is continuous from a connection portion of the tube 23 for the guide wire lumen beyond a connection portion with the connector unit 27.

In the inside of a tubular lumen of a catheter sheath 24, there is inserted an imaging core 420 approximately over the full length of the catheter sheath 24. On the distal side (the side on which the tube 23 for guide wire lumen is provided) of the imaging core 25, there is provided a housing 21 which is provided with a transmitting & receiving unit for transmitting & receiving the measurement light. Also, at the imaging core 25, there is provided also a drive shaft 22 for transmitting a drive force for rotating the housing 21.

The connector unit 27 is composed of a hand-side portion 27a constituted integrally at the proximal end of the catheter sheath 24 and a connection portion 27b constituted integrally at the proximal end of the drive shaft 22.

At the boundary portion between the hand-side portion 27a and the catheter sheath 24, there is provided an anti-kink protector 26. Thus, a predetermined rigidity can be maintained and it is possible to prevent a bending (kink) caused by a rapid change. At the proximal end of the connection portion 27b, there is provided a connection connector 30 and the connection connector 30 is constituted so as to be connectable with the adapter unit 40 of the scanner & pull-back unit 12.

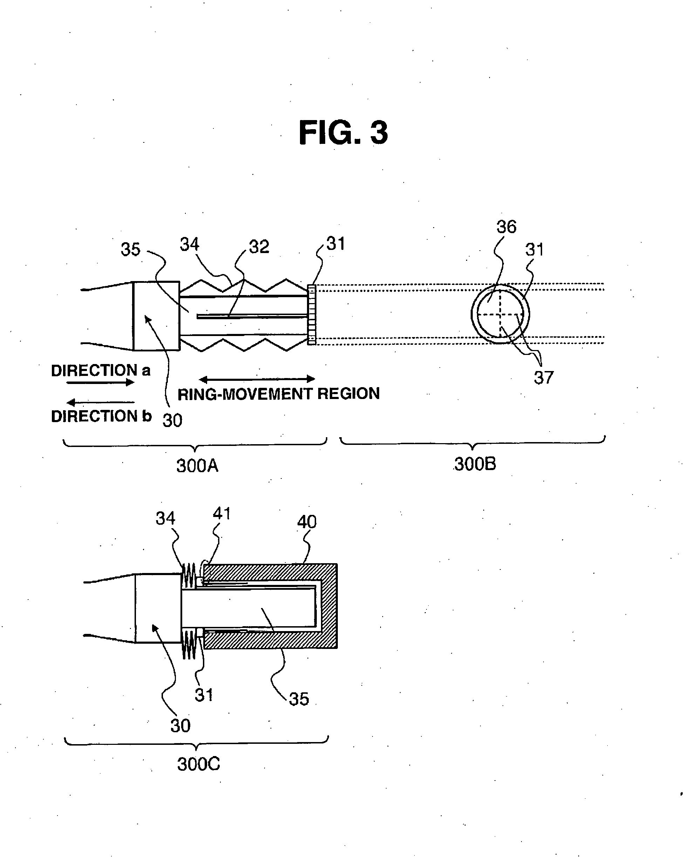

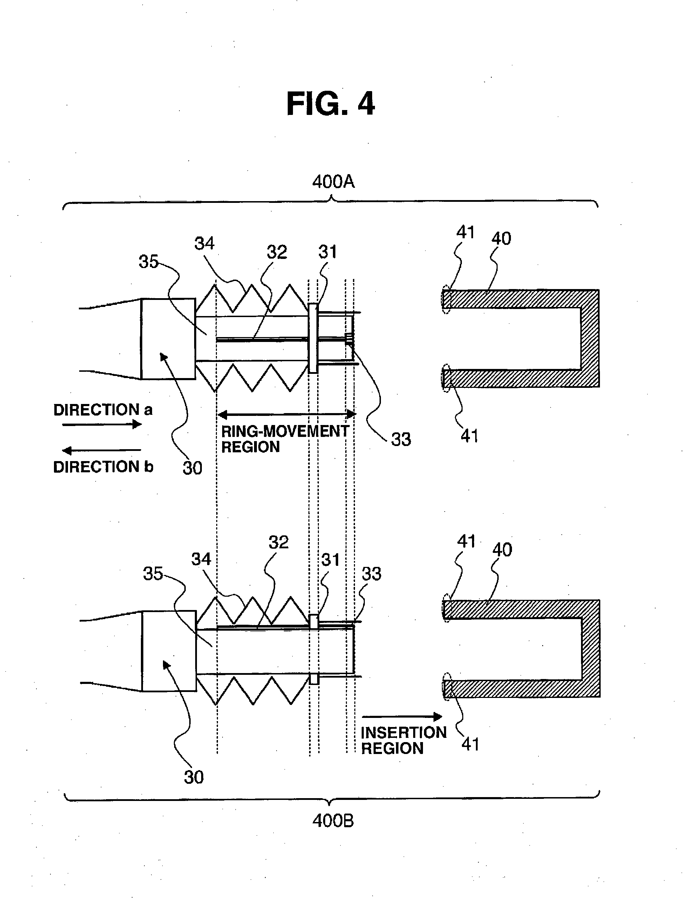

Here, there will be explained one example of an outlined constitution of the connection connector unit 30 by using FIG. 3 and FIG. 4. A reference numeral 300A in FIG. 3 shows one example of a cross-sectional constitution of the connection connector unit 30, and the reference numeral 300B in FIG. 3 shows one example of the outlined constitution in a case in which the ring 31 shown within the reference numeral 300A in FIG. 3 is seen from a b-direction, and a reference numeral 300C in FIG. 3 shows a view in which the connection connector unit 30 is connected (coupled) with the adapter unit 40. Also, a reference numeral 400A in FIG. 4 shows one example of the cross-sectional constitution in a case in which the ring 31 is moved by a predetermined distance in the b-direction from a state shown by the reference numeral 300A in FIG. 3, and a reference numeral 400B in FIG. 4 shows one example of the cross-sectional constitution of the connection connector unit 30, which is seen from the lateral direction in a case in which a state shown by the reference numeral 400A in FIG. 4 is supposed to be a drawing which is seen from the upper direction.

Note that an a-direction shown in FIG. 3 and FIG. 4 indicates an insertion direction (first direction) when the connection connector unit 30 (optical probe unit 11) is inserted into the adapter unit 40, and the b-direction shown in FIG. 3 and FIG. 4 indicates a direction (second direction: direction opposite to the first direction) when the connection connector unit 30 (optical probe unit 11) is released from a state of being connected to the adapter unit 40.

At the connection connector unit 30, there is provided a connector 35 which is actually inserted into the adapter unit 40. Note that the connector 35 relating to this exemplified embodiment is formed in a cylindrical shape.

Here, for the connector 35, there are provided a ring 31, a rail 32, a stopper 33, a side surface cover 34 and a connection-end surface cover 36.

The ring 31 is provided at the outer circumference of the connector 35 and is constituted to have a predetermined thickness in the direction perpendicular to the peripheral surface of the connector 35.The ring 31 moves along the rail 32. More specifically, the length along the a-direction of the rail 32 becomes a movement region of the ring 31.

The rail 32 is provided along the a-direction (insertion direction of the connector) on the connector 35 and play a role of guiding the ring 31. As shown by the reference numeral 400B in FIG. 4, the rail 32 is formed in a convex shape.

The stopper 33 is provided at the end portion (on the connection end surface side) of the connector 35 and plays a role of preventing the ring 31 from dropping from the connector 35. The ring 31 usually receives a press force directed toward the a-direction by the side surface cover 34 and lies in a state of being attached to the stopper 33.

Here, the connector 35 is covered by cover members. More specifically, the connector 35 is covered by the side surface cover 34 and a connection-end surface cover 36.

The side surface cover 34 covers the whole side surface portion of the connector 35, prevents contamination of the connector 35, and concurrently, plays a role of preventing the liquid from intruding. The side surface cover 34 is constituted by a material having flexibility or elasticity such as, for example, PE, PP and silicone rubber.

The side surface cover 34 has a bellow shape and is constituted to be contractible toward the b-direction. With regard to the side surface cover 34, there is provided a elastic member such as a spring or the like in the inside thereof and a press force directed toward the a-direction is applied to the ring 31. The side surface cover 34 becomes a state shown by the reference numeral 300A in FIG. 3 before the connection and becomes a state of being contracted as shown by the reference numeral 300C in FIG. 3 in a case in which the connector 35 is inserted into the adapter unit 40.

As shown by the reference numeral 300B in FIG.3, for the ring 31, there is provided the connection-end surface cover 36 in which perforations are provided in the vertical and horizontal directions. The connection-end surface cover 36 is constituted by a thin film such as, for example, PE, PP, PET, PVC and OPS.

The connection-end surface cover 36 is broken by the connector 35, along with the movement of the ring 31 in the b-direction, by a press force directed toward the a-direction when the connector 35 is inserted into the adapter unit 40. More specifically, in a case in which the connector 35 is inserted into the adapter unit 40, as shown by the reference numeral 300C in FIG. 3, the ring 31 is pressed toward the b-direction by the wall surface (press portion 41) of the insertion port (opening of adapter unit 40) and the connection end-surface cover 36 is broken along the perforations by the press force thereof. Also, at that time, the side surface cover 34 is contracted in the b-direction and the connector 35 is inserted into the adapter unit 40. Note that the optical probe unit 11 is usually used once and then thrown away, so that it is not necessary for the aforementioned connection-end surface cover 36 to return to the original state and there is no problem in particular even if it is broken.

As explained above, according to the exemplified embodiment 1, the whole surface of the connector 35 is covered by the side surface cover 34 and the connection-end surface cover 36, so that the connector 35 is never exposed before the connection between the connection connector unit 30 and the adapter unit 40.

Therefore, on an occasion of the connection between the connector unit 30 and the adapter unit 40, the connector 35 never becomes dirty even if the operator grabs whatever portion at the connection connector unit 30. Further, even if the side surface cover 34 becomes wet before the connection, it never happens that the inside of the adapter unit 40 is got wet.

Also, during the time when the connection connector unit 30 and the adapter unit 40 are connected, the side surface cover 34 having a bellow shape covers the connector 35 in a contracted state, so that the connector 35 is never exposed to the outside. Even on an occasion of the removal of the connection, the side surface cover 34 returns to the original state, so that it never happens that the operator touches the connector 35 directly.

Furthermore, the connection-end surface cover 36 has a structure in which it is broken when inserting the connection connector unit 30 thereinto, so that on an occasion of the connection between the connection connector unit 30 and the adapter unit 40, the operator can accomplish the insertion directly by a single hand without touching the connection end surface or the like. Thus, it never happens that the connector 35 is got dirty and in addition, the connector 35 can be inserted by a single hand, so that also the usability thereof is improved.

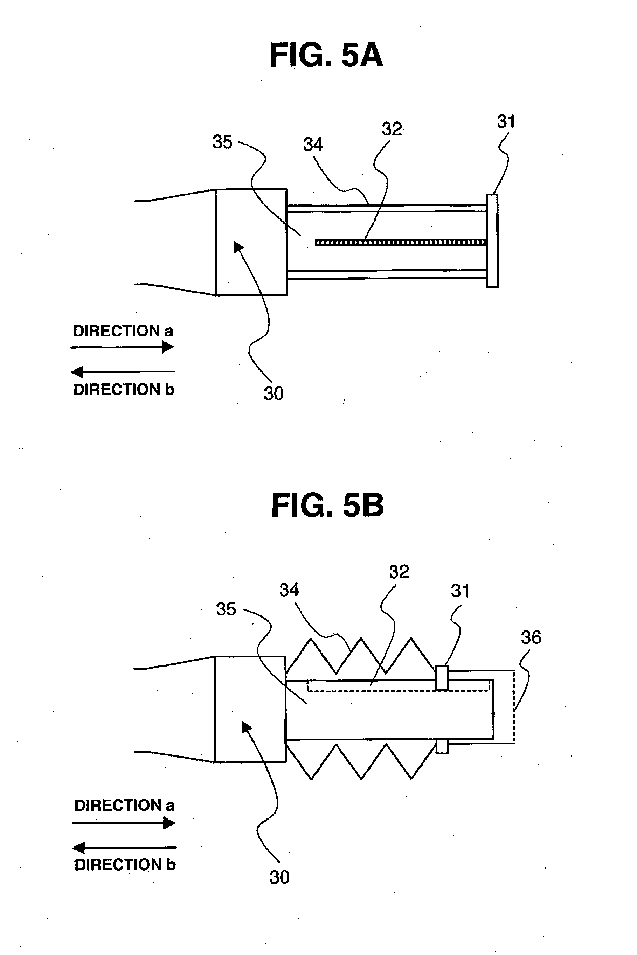

Note that, in the explanation mentioned above, the explanation was carried out by citing, as an example, a case in which an elastic member is installed in the side surface cover 34 and in which the aforementioned cover 34 returns to the original state when removing the connection between the connection connector unit 30 and the adapter unit 40, but it is not always necessary to employ such a constitution. As mentioned above, the optical probe unit 11 is usually used once and then thrown away, so that it is not always necessary to employ a configuration in which the elastic member is installed in the side surface cover 34 and the original state is restored when removing the connection. In this case, it is allowed for the side surface cover 34 not to be constituted by the bellow shape which is, for example, shown in FIG. 5A.

Also, in the explanation mentioned above, the explanation was carried out by citing a case of providing the stopper 33 as an example, but it is not always necessary to provide the stopper 33. For example, as shown in FIG. 5B, it is allowed for the rail 32 to have a role of the stopper by employing a constitution in which the rail 32 is formed in a concave shape. Furthermore, in the explanation mentioned above, the movement of the ring 31 is guided by the rail 32, but this rail 32 is not an indispensable element either and can be omitted.

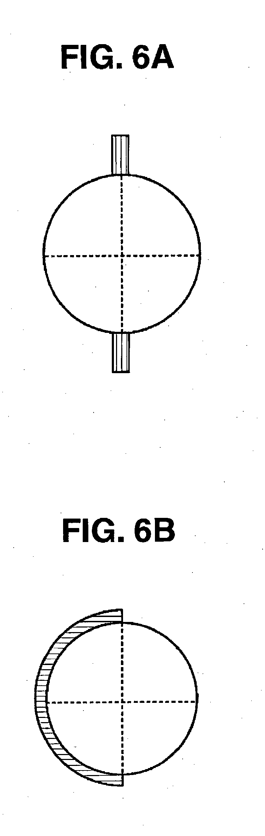

Also, in the explanation mentioned above, the ring 31 has a circular shape which covers the whole outer circumference of the connector 35, but it is enough if there is employed a configuration in which the side surface cover 34 can be contracted in the b-direction by a press force which is received from the wall surface (press portion 41) forming an opening of the adapter unit 40 and it is not always necessary to employ such a shape. FIG. 6A and FIG. 6B are views showing an outlined constitution of the ring 31 seen from the same direction as that of the reference numeral 300B in FIG. 3, in which as shown in FIG. 6A or FIG. 6B, it is allowed for the ring 31 to be constituted by having thickness only at a portion of the outer circumference of the connector 35.

Also, it is allowed for the side surface cover 34 and the connection-end surface cover 36 to be constituted as an integrated cover by using the same material and also, it is allowed for them to be constituted by different materials respectively.

### (Exemplified Embodiment 2)

Next, there will be explained an exemplified embodiment 2. Note that the apparatus constitution and the like are similar to those of FIG. 1 and FIG. 2 which were used for explaining the exemplified embodiment 1, so that the explanations thereof will be omitted and here, there will be focused on explaining difference points therebetween.

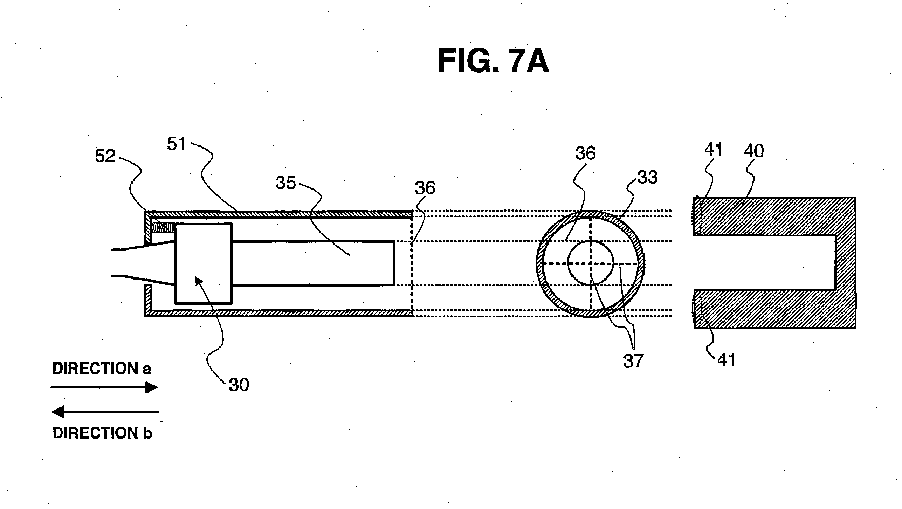

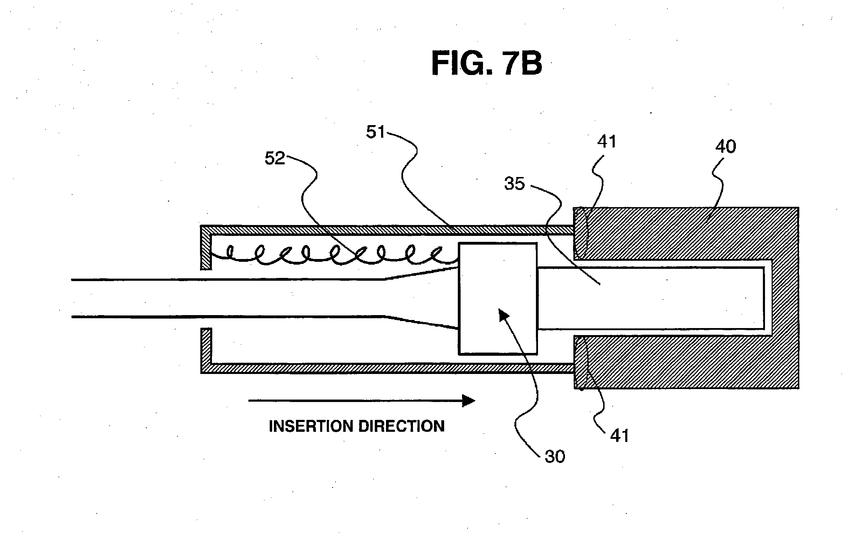

FIG. 7A and FIG. 7B are views showing one example of a constitution of a connection connector unit 30 relating to the exemplified embodiment 2. FIG. 7A shows one example of a cross-sectional constitution of the connection connector unit 30 seen from a predetermined direction, and FIG. 7B shows a view in which the connection connector unit 30 is connected (coupled) with the adapter unit 40.

Note that, similarly as the exemplified embodiment 1, the a-direction shown in FIG. 7A and FIG. 7B shows the insertion direction (first direction) when the connection connector unit 30 (optical probe unit 11) is inserted into the adapter unit 40, and the b-direction shown in FIG. 7A and FIG. 7B shows the direction when the connection connector unit 30 (optical probe unit 11) is released from a state of being connected to the adapter unit 40 (second direction: direction opposite to the first direction).

At the connection connector unit 30 relating to the exemplified embodiment 2, there are actually provided a cover 51 and an elastic member 52 other than the connector 35 which is inserted into the adapter unit 40. Note that the same reference numerals are applied to the same constitutions as those of FIG. 3 and FIG. 4, and there sometimes happens a case in which the explanations thereof will be omitted.

Here, similarly as the exemplified embodiment 1, the connector 35 is covered by cover members having predetermined distances in the perpendicular directions from the respective portions of the connector.
More specifically, the connector 35 is covered by the cover 51 and the connection end surface cover 36.

The cover 51 covers the whole side surface portion of the connector 35, and avoid the dirt of the connector 35 and concurrently, plays a role of preventing the liquid from intruding. The cover 51 has a tubular shape and is constituted to be movable toward the b-direction. On the inner wall of the cover 51, there is provided an elastic member 52 such as a spring, a rubber or the like. Note that, at the position abutting the connection end surface of the connector 35, similarly as the exemplified embodiment 1, there is provided a connection-end surface cover 36 for which perforations are provided in the vertical and horizontal directions.

Here, the cover 51 is pressed toward the b-direction by the wall of the end surface (press portion 41) of the adapter unit 40 when the connector 35 is inserted into the adapter unit 40. More specifically, in a case in which the connector 35 is inserted into the adapter unit 40, as shown in FIG. 7B, the cover 51 is pressed toward the b-direction and caused by a press force thereof, the connection-end surface cover 36 is broken along the perforations and concurrently, the cover 51 moves toward the b-direction and the connector 35 is inserted into the adapter unit 40.

Also, in a case in which the connector 35 is removed from the adapter unit 40, the cover 51 returns to the original position by an action of the elastic member 52. Note that, as mentioned above, the optical probe unit 11 is usually used once and then thrown away, so that it is not always necessary to provide the elastic member 52.

As explained above, according to the exemplified embodiment 2, the whole surface of the connector 35 is covered by the cover 51 and the connection-end surface cover 36, so that the connector 35 is never exposed before the connection between the connection connector unit 30 and the adapter unit 40. Also in this case, an effect can be obtained similarly as the exemplified embodiment 1.

Note that it is allowed for the cover 51 and the connection-end surface cover 36 to be constituted as an integrated cover by using the same material and also, it is allowed for them to be constituted by different materials respectively.

The above descriptions relate to examples of representative exemplified embodiments of the present invention, but the present invention is not limited by the exemplified embodiments shown in the abovementioned descriptions and the drawings, and the present invention can be practiced by applying modifications appropriately within the scope without departing from the gist thereof.

For example, in the exemplified embodiments 1 and 2 mentioned above, the explanation was carried out by citing, as an example, a case in which perforations are formed on the connection-end surface cover 36 and the connection-end surface cover 36 is broken along the aforementioned perforations when the connector 35 is inserted into the adapter unit 40, but for the connection-end surface cover 36, it is not always necessary to form the perforations. More specifically, in this exemplified embodiment, it is allowed to employ any constitution if the connection-end surface cover 36 is broken when the connector 35 is inserted into the adapter unit 40. For example, it is allowed for the connection-end surface cover 36 to be constituted by a thin and breakable material without providing any perforations. Also, it is allowed to employ a constitution in which the end portion of the connector 35 is formed in a sharpened shape and depending on that shape, the connection-end surface cover 36 is to be broken.

Also, in the exemplified embodiments 1 and 2 mentioned above, the explanation was carried out for one example of the probe by citing, as an example, an optical probe unit connected to the imaging apparatus for diagnosis using light, but it is not limited by this aspect. For example, it is also possible to apply the constitution mentioned above with respect to a probe unit connected to an imaging apparatus for diagnosis which images (generates and displays) a tomographic image of the blood vessel by utilizing ultra-sound.

The present invention is not to be limited by the abovementioned exemplified embodiments, and various changes and modifications can be employed without departing from the scope of the present invention. Therefore, in order to publicly disclose the scope of the present invention, the following claims will be attached.

## Claims

1. A probe (11) to be inserted inside a body-cavity, **characterized by** comprising:
a connector (35) inserteable into an adapter unit (40) provided in an imaging apparatus for diagnosis (10) which generates and displays a tomographic image inside the body-cavity and whose end portion constitutes a connection end surface;
a ring (31) provided on the connection end surface side of the connector (35) concurrently, having a predetermined thickness in a direction perpendicular to the peripheral surface of the connector and movable toward a second direction expressing a direction opposite to a first direction expressing the direction toward which the probe (11) is inserted from the connection end surface side of the connector;
a first cover engaged with the ring, (31) constituting a surface perpendicular to the first direction and covering the connection end surface side of the connector (35); and
a second cover constituted in a contractible manner toward the second direction, engaged with the ring, (31) stretched toward the second direction and covering the connector (35), wherein
it is constituted such that:
the ring (31) receives a press force directed from a wall surface formed with an opening of the adapter unit toward the second direction and moves from the connection end surface side of the connector (35) toward the second direction at the time of insertion of the connector (35) into the adapter unit (40),
the second cover contracts toward the second direction with the movement of the ring (31) toward the second direction, and
the first cover is to be broken by the connector's press force directed toward the first direction in a case in which the ring (31) moves toward the second direction.

2. The probe according to claim 1, **characterized in that** the second cover returns from the contracted state to the original state in a case in which the connector (35) is released from the state in which the connector is connected to the adapter unit (40).

3. The probe according to claim 1, **characterized in that**
the second cover has a bellow shape and presses the ring (31) toward the first direction; and
the connector includes:
a stopper (33) for preventing the ring from dropping from the connector toward the first direction, and
a rail (32) for guiding the movement of the ring (31) from the connection end surface side of the connector toward the second direction.

4. An imaging apparatus for diagnosis, **characterized by** comprising:
a probe (11) according to any of claims 1 to 3 to be inserted inside a body-cavity,
an adapter apparatus including an adapter unit (40) connecteable with the probe (11) and giving and receiving various kinds of signals with respect to the probe through the adapter unit (40), and
a control unit (18) for controlling the adapter unit (40) and for generating a tomographic image inside the body-cavity.

## Patentansprüche

1. Sonde (11) zum Einfügen in eine Körperhöhle, **dadurch gekennzeichnet, dass** sie Folgendes umfasst:
ein Verbindungsstück (35), das in eine Adaptereinheit (40) einfügbar ist, die in einem Bildgebungsgerät zur Diagnose (10) bereitgestellt wird, das ein tomografisches Bild im Innern der Körperhöhle generiert und anzeigt und dessen Endabschnitt eine Verbindungsendfläche bildet;
einen Ring (31), der auf der Seite der Verbindungsendfläche des Verbindungsstücks (35) gleichzeitig bereitgestellt wird und eine vorbestimmte Dicke in einer Richtung aufweist, die zur Umfangsfläche des Verbindungsstücks rechtwinklig ist, und in eine zweite Richtung bewegbar ist, die eine Richtung darstellt, die einer ersten Richtung entgegengesetzt ist, welche die Richtung darstellt, in welche die Sonde (11) von der Seite der Verbindungsendfläche des Verbindungsstücks aus eingefügt wird;
eine erste Abdeckung, die mit dem Ring (31) in Eingriff steht und eine Oberfläche bildet, die zu der ersten Richtung rechtwinklig ist und die Seite der Verbindungsendfläche des Verbindungsstücks (35) abdeckt; und
eine zweite Abdeckung, die in die zweite Richtung zusammendrückbar gestaltet ist, mit dem Ring (31) in Eingriff steht, in der zweiten Richtung gedehnt wird und das Verbindungsstück (35) abdeckt, wobei
sie derart gestaltet ist, dass:
der Ring (31) eine Andrückkraft aufnimmt, die von einer Wandfläche, die mit einer Öffnung der Adaptereinheit gebildet ist, in die zweite Richtung gerichtet ist, und sich von der Seite der Verbindungsendfläche des Verbindungsstücks (35) in die zweite Richtung bewegt, wenn das Verbindungsstück (35) in die Adaptereinheit (40) eingefügt wird,
die zweite Abdeckung durch die Bewegung des Rings (31) in die zweite Richtung in die zweite Richtung zusammengedrückt wird, und
die erste Abdeckung dazu gedacht ist, durch die Andrückkraft des Verbindungsstücks zerbrochen zu werden, die in die erste Richtung gerichtet ist, für den Fall, dass sich der Ring (31) in die zweite Richtung bewegt.

2. Sonde nach Anspruch 1, **dadurch gekennzeichnet, dass** die zweite Abdeckung von dem zusammengedrückten Zustand in den ursprünglichen Zustand zurückkehrt für den Fall, dass das Verbindungsstück (35) aus dem Zustand gelöst wird, in dem das Verbindungsstück mit der Adaptereinheit (40) verbunden ist.

3. Sonde nach Anspruch 1, **dadurch gekennzeichnet, dass**
die zweite Abdeckung eine Faltenbalgform aufweist und den Ring (31) in die erste Richtung drückt; und
das Verbindungsstück Folgendes umfasst:
einen Stöpsel (33), um zu verhindern, dass der Ring aus dem Verbindungsstück in die erste Richtung fällt, und
eine Schiene (32) zum Führen der Bewegung des Rings (31) von der Seite der Verbindungsendfläche des Verbindungsstücks in die zweite Richtung.

4. Bildgebungsgerät zur Diagnose, **dadurch gekennzeichnet, dass** es Folgendes umfasst:
eine Sonde (11) nach einem der Ansprüche 1 bis 3, die in eine Körperhöhle einzufügen ist,
ein Adaptergerät, das eine Adaptereinheit (40) umfasst, die mit der Sonde (11) zu verbinden ist und diverse Signale mit Bezug auf die Sonde über die Adaptereinheit (40) abgibt und empfängt, und
eine Steuereinheit (18) zum Steuern der Adaptereinheit (40) und zum Generieren eines tomografischen Bildes im Innern der Körperhöhle.

## Revendications

1. Sonde (11) destinée à être insérée dans une cavité corporelle, **caractérisée en ce qu'**elle comprend :
un connecteur (35) pouvant être inséré dans une unité d'adaptateur (40) prévue dans un appareil d'imagerie pour diagnostic (10) qui génère et affiche une image tomographique à l'intérieur de la cavité corporelle et dont la partie d'extrémité constitue une surface d'extrémité de connexion ;
une bague (31) placée simultanément du côté de la surface d'extrémité de connexion du connecteur (35), ayant une épaisseur prédéterminée dans une direction perpendiculaire à la surface périphérique du connecteur et mobile vers une deuxième direction exprimant une direction opposée à une première direction exprimant la direction vers laquelle la sonde (11) est insérée depuis le côté de la surface d'extrémité de connexion du connecteur ;
un premier couvercle en prise avec la bague (31) constituant une surface perpendiculaire à la première direction et couvrant le côté de la surface d'extrémité de connexion du connecteur (35) ; et
un deuxième couvercle constitué de manière rétractable vers la deuxième direction, en prise avec la bague (31), étiré vers la deuxième direction et couvrant le connecteur (35), et constitué de telle manière que :
la bague (31) reçoit une force de pression dirigée d'une surface de paroi munie d'une ouverture de l'unité d'adaptateur vers la deuxième direction et se déplace du côté de la surface d'extrémité de connexion du connecteur (35) vers la deuxième direction au moment de l'insertion du connecteur (35) dans l'unité d'adaptateur (40),
le deuxième couvercle se contracte vers la deuxième direction avec le déplacement de la bague (31) vers la deuxième direction, et
le premier couvercle est destiné à être cassé par la force de pression du connecteur dirigée vers la première direction dans le cas où la bague (31) se déplace vers la deuxième direction.

2. Sonde selon la revendication 1, **caractérisée en ce que** le deuxième couvercle revient de l'état contracté à l'état d'origine dans le cas où le connecteur (35) est libéré de l'état dans lequel le connecteur est connecté à l'unité d'adaptateur (40).

3. Sonde selon la revendication 1, **caractérisée en ce que** le deuxième couvercle a la forme d'un soufflet et pousse la bague (31) vers la première direction ; et
le connecteur comprend :
une butée (33) pour empêcher la bague de sortir du connecteur vers la première direction, et
un rail (32) pour guider le déplacement de la bague (31) du côté de la surface d'extrémité de connexion du connecteur vers la deuxième direction.

4. Appareil d'imagerie pour diagnostic, **caractérisé en ce qu'**il comprend :
une sonde (11) selon l'une quelconque des revendications 1 à 3, destinée à être insérée à l'intérieur d'une cavité corporelle,
un appareil adaptateur comprenant une unité d'adaptateur (40) pouvant être connectée à la sonde (11) et qui donne et reçoit diverses sortes de signaux relativement à la sonde par l'intermédiaire de l'unité d'adaptateur (40), et
une unité de commande (18) pour commander l'unité d'adaptateur (40) et pour générer une image tomographique à l'intérieur de la cavité corporelle.
